(19) Europäisches Patentamt
European Patent Office
Office européen des brevets

(11) Publication number : **0 541 286 A1**

# (12) EUROPEAN PATENT APPLICATION

(21) Application number : **92309842.0**

(51) Int. Cl.[5] : **C07C 2/16,** B01J 37/00

(22) Date of filing : **27.10.92**

(30) Priority : **04.11.91 US 787164**
**09.12.91 US 803811**

(43) Date of publication of application :
**12.05.93 Bulletin 93/19**

(84) Designated Contracting States :
**DE FR GB**

(71) Applicant : **TEXACO CHEMICAL COMPANY**
**3040 Post Oak Boulevard**
**Houston, Texas 77056 (US)**

(72) Inventor : **Sanderson, John Ronald**
**15290 Faubion Trail**
**Leander, TX 78641 (US)**
Inventor : **Knifton, John Frederick**
**10900 Catskill Trail**
**Austin, TX 78727 (US)**
Inventor : **Larkin, John Michael**
**12414 Dorsett Road**
**Austin, TX 78727 (US)**
Inventor : **Marquis, Edward Thomas**
**9004 Collinfield Drive**
**Austin, TX 78758 (US)**

(74) Representative : **Green, Mark Charles et al**
**Urquhart-Dykes & Lord, 91 Wimpole Street**
**London W1M 8AH (GB)**

## (54) Process for oligomerizing olefins using sulfate-activated catalysts.

(57) A process for the preparation of oligomers comprising oligomerizing a linear olefin containing from 10 to 24 carbon atoms in the presence of a catalyst, characterised in that said catalyst is a sulfate activated Group IV oxide or molecular sieve.

EP 0 541 286 A1

The invention relates to the preparation of synthetic lubricant base-stocks, and more particularly to synthetic lubricant base stocks made by oligomerizing linear olefins.

Synthetic lubricants are prepared from man-made base stocks having uniform molecular structures and, therefore, well-defined properties that can be tailored to specific applications. Mineral oil base stocks, on the other hand, are prepared from crude oil and consist of complex mixtures of naturally occurring hydrocarbons. The greater uniformity of synthetic lubricants generally results in superior performance properties. For example, synthetic lubricants have excellent thermal stability. As automobile engines are reduced in size to save weight and fuel, they run at higher temperatures and therefore require a more thermally stable oil. Because lubricants made from synthetic base stocks have such properties as excellent oxidative/thermal stability, very low volatility, and good viscosity indices over a wide range of temperatures, they offer better lubrication, and permit longer drain intervals with less oil vaporization loss between oil changes, than mineral oil base stocks.

Synthetic base stocks may be prepared by oligomerizing internal and alpha-olefin monomers to form a mixture of dimers, trimers, tetramers, and pentamers, with minimal amounts of higher oligomers. The unsaturated oligomer products are then hydrogenated to improve their oxidative stability. The resulting synthetic base stocks have uniform isoparaffinic hydrocarbon structures similar to high quality paraffinic mineral base stocks, but have the superior properties mentioned above, because of their greater uniformity.

Synthetic base stocks are produced in a broad range of viscosity grades. It is common practice to classify the base stocks by their viscosities, measured in centistokes (cSt) at 100°C. Those base stocks with viscosities less than or equal to about 4 cSt are commonly referred to as "low viscosity" base stocks, whereas base stocks having a viscosity in the range of around 40 to 100 cSt are commonly referred to as "high viscosity" base stocks. Base stocks having a viscosity of about 4 to about 8 cSt are referred to as "medium viscosity" base stocks. The low viscosity base stocks generally are recommended for low temperature applications. Higher temperature applications, such as motor oils, automatic transmission fluids, turbine lubricants, and other industrial lubricants, generally require higher viscosities, such as those provided by medium viscosity base stocks (i.e. 4 to 8 cSt grades). High viscosity base stocks are used in gear oils and as blending stocks. 1 centistoke is equal to 1 x $10^{-6}m^2/sec$.

The viscosity of a base stock is determined by the length of the oligomer molecules formed during the oligomerization reaction. The degree of oligomerization is affected by the catalyst and reaction conditions employed during the oligomerization reaction. The length of the carbon chain of the monomer starting material also has a direct influence on the properties of the oligomer products. Fluids prepared from short-chain monomers tend to have low pour points and moderately low viscosity indices, whereas fluids prepared from long-chain monomers tend to have,, moderately low pour points and higher viscosity indices. Oligomers prepared from long-chain monomers generally are more suitable for use as medium viscosity synthetic lubricant base stocks than those prepared from shorter-chain monomers.

One known approach to oligomerizing long-chain olefins to prepare synthetic lubricant base stocks is to contact the olefin with boron trifluoride together with a promotor at a reaction temperature sufficient to effect oligomerization of the olefin. See, for example U.S. Patent Nos. 4,400,565; 4,420,646; 4,420,647; and 4,434,308. However, boron trifluoride gas ($BF_3$) is a pulmonary irritant, and breathing the gas or fumes formed by hydration of the gas with atmospheric moisture poses hazards preferably avoided. In addition, the disposal/neutralization of $BF_3$ raises environmental concerns. Thus, a method for oligomerizing long-chain olefins using a less hazardous catalyst would be a substantial improvement in the art.

It has now been found that a high conversion of olefin to oligomer may be obtained by contacting the olefin with a sulfate activated catalyst wherein said catalyst is either a molecular sieve or a Group IV oxide.
such as sulfate-activated titanium dioxide or sulfate-activated zirconium dioxide.

When using sulfate-activated molecular sieves, it has been found that an even higher conversion of olefin to oligomer may be obtained by contacting the olefin with a catalyst prepared by sulfate-activating a molecular sieve that has previously been treated to generate additional Bronsted acid sites. A higher conversion of olefin to oligomer is observed using a sulfate-activated molecular sieve than is observed using the same molecular sieve without sulfate-activation.

The process of the present invention results in a high percentage of dimers, i.e., a high dimer to trimer ratio. A high proportion of dimers is often desirable when preparing a synthetic lubricant base stock from olefins having about 14 or more carbon atoms. In the absence of the high dimer to trimer ratio obtained using the present invention, a synthetic lubricant base stock prepared from olefins having about 14 or more carbon atoms would contain a higher percentage of high molecular weight oligomers and may have too great a viscosity for some applications. In addition to being excellent catalysts, the sulfate-activated catalysts used in the present invention are less hazardous and more easily handled than $BF_3$.

The invention relates to a process for the preparation of oligomers, comprising oligomerizing a linear olefin containing from 10 to 24 carbon atoms in the presence of a sulfate-activated catalyst wherein said catalyst is

either a molecular sieve or a Group IV oxide. The invention further relates to a process for the preparation of oligomers, comprising oligomerizing a linear olefin containing from 10 to 24 carbon atoms in the presence of a sulfate activated catalyst wherein said catalyst is either a molecular sieve or a Group IV oxide at a temperature in the range of 50°C to 300°C and at a pressure of atmospheric to 6895 kPa gauge pressure (gp) (1000 psig). The invention also relates to a process for the preparation of oligomers, comprising oligomerizing a linear olefin containing from 14 to 18 carbon atoms in the presence of a catalytically effective amount of a Group IV oxide selected from titanium dioxide and zirconium dioxide, which Group IV oxide has been (1) treated with a sulfur-containing compound selected from ammonium sulfate, ammonium hydrogen sulfate, sulfuric acid, sulfur dioxide, sulfur trioxide, and hydrogen sulfide and (2) calcined at a temperature greater than 500°C, and wherein the olefin is oligomerized at a temperature in the range of 120°C to 250°C and at a pressure of atmospheric to 6895kPa gp (1000 psig).

The invention further relates to a process for the preparation of oligomers, comprising contacting linear olefins containing from 10 to 24 carbon atoms with a catalytically effective amount of sulfate-activated crystalline aluminosilicate molecular sieves, at a temperature in the range of 50°C to 300°C and at a pressure of atmospheric to 6895kPa gp (1000 psig). The invention also relates to a process for the preparation of oligomers, comprising the steps of (a) oligomerizing linear olefins containing from 14 to 18 carbon atoms in the presence of a catalytically effective amount of crystalline aluminosilicate molecular sieves having enhanced Bronsted acidity, which crystalline aluminosilicate molecular sieves have been sulfate-activated by treatment with a sulfate-containing compound and calcined at a temperature in the range of about 500° to 800°C, wherein the olefin is oligomerized at a temperature in the range of 120°C to 250°C and at a pressure of atmospheric to 6895kPa gp (1000 psig), and (b) recovering oligomers of said linear olefins.

The olefins may be co-oligomerized with propylene, 1,3-diisopropylene benzene or alpha-methyl styrene, or with vinylidene olefins having 10 to 24 carbon atoms. This is described more fully in EP-A-449453.

The olefin monomer feed stocks used in the present invention may be selected from compounds comprising (1) alpha-olefins having the formula $R''CH=CH_2$, where R″ is an alkyl radical of 8 to 22 carbon atoms, and (2) internal olefins having the formula RCH=CHR′, where R and R′ are the same or different alkyl radicals of 1 to 21 carbon atoms, provided that the total number of carbon atoms in any one olefin shall be within the range of 10 to 24, inclusive. A preferred range for the total number of carbon atoms in any one olefin molecule is 12 to 18, inclusive, with an especially preferred range being 14 to 16, inclusive. Mixtures of internal and alpha-olefins may be used, as well as mixtures of olefins having different numbers of carbon atoms, provided that the total number of carbon atoms in any one olefin shall be within the range of 10 to 24, inclusive. The alpha and internal-olefins to be oligomerized in this invention may be obtained by processes of the prior art and are commercially available.

The oligomerization reaction may be represented by the following general equation:

$$nC_mH_{2m} \xrightarrow{\text{catalyst}} C_{mn}H_{2mn}$$

where n represents moles of monomer and m represents the number of carbon atoms in the monomer. Thus, the oligomerization of 1-decene may be represented as follows:

$$nC_{10}H_{20} \xrightarrow{\text{catalyst}} C_{10n}H_{20n}$$

The reaction occurs sequentially. Initially, olefin monomer reacts with olefin monomer to form dimers. Some of the dimers that are formed then react with additional olefin monomers to form trimers, and so on. This results in an oligomer product distribution that varies with reaction time. As the reaction time increases, the olefin monomer conversion increases, and the selectivities for the heavier oligomers increase. Generally, each resulting oligomer contains one double bond.

In one embodiment of the present invention, the catalysts are sulfate-activated molecular sieves. The terms "sulfate-activated" and "sulfate-activating" refer to the step of treating molecular sieves with one or more sulfate-containing compounds to form sulfate radicals on the oxide or molecular sieves and thus provide a sulfate-activated surface on the substrate, as further described below. Molecular sieves suitable for sulfate-activation may be crystalline aluminosilicates, or may be essentially alumina-free silicates, such as, for example, silicalite, chromia silicates, ferrosilicates, and others.

Preferred molecular sieves are crystalline aluminosilicates having a three-dimensional interconnecting

network of silica and alumina tetrahedra, belonging to a class of minerals known as zeolites. These preferred molecular sieves are complex, crystalline inorganic polymers based on an infinitely extending framework of $AlO_4$ and $SiO_4$ tetrahedra that are linked to each other by oxygen. This framework contains channels, or interconnected voids, that may be occupied by cations and by water molecules. The water molecules may be removed (reversibly), generally by the application of heat, which leaves intact a crystalline host structure permeated by micropores that may amount to 50 % of the crystals by volume. Such molecular sieves may be represented by the following formula:

$$M_{2/a}O.Al_2O_3.ySiO_2.wH_2O$$

where M represents a cation, a is the valence of the cation, y is about 2 or greater, and w represents the number of water molecules per unit cell. Further description of various aluminosilicate molecular sieves may be found in Kirk-Othmer, Encyclopedia of Chemical Technology, 3d. ed., vol. 15, pp. 638-669 (1981), incorporated herein by reference.

Examples of suitable commercially available molecular sieves are the Aldrich molecular sieves 3A (0.6 $K_2O$ : 0.40 $Na_2O$ : 1 $Al_2O_3$ : 2.0 $\pm$ 0.1 $SiO_2$ : x $H_2O$), having a pore diameter of about 3 Å; 4A (1 $Na_2O$ : 1 $Al_2O_3$ : 2.0 $\pm$ 0.1 $SiO_2$ : x $H_2O$), having a pore diameter of about 4 Å; 5A (0.80 CaO : 0.20 $Na_2O$ : 1 $Al_2O_3$ : 2.0 $\pm$ 0.1 $SiO_2$ : x $H_2O$), having a pore diameter of about 5 Å; and 13X (1 $Na_2O$ : 1 $Al_2O_3$ : 2.8 $\pm$ 0.2 $SiO_2$ : x $H_2O$), having a pore diameter of about 10 Å.

Preferably, the molecular sieves to be sulfate-activated have enhanced Bronsted acidity, i.e., they have been treated to generate additional Bronsted acid sites prior to being sulfate-activated . Bronsted acidity may be introduced into the molecular sieves by decomposition of the ammonium ion-exchanged form, by hydrogen ion exchange (i.e., by washing with a mineral acid), or by hydrolysis of a zeolite containing transition metal cations.

Decomposition: $Z\text{-}NH_4 \text{ ------> } Z\text{-}H + NH_3$

Acid Treatment: $Z\text{-}Na + H^+ \text{ ------> } Z\text{-}H + Na^+$

Hydrolysis: $Z\text{-}Cr^{+++} + H_2O \text{ ------> } Z\text{-}Cr(OH)^{++} + H^+$

Suitable commercially available acid-washed molecular sieves include the Aldrich acid-washed molecular sieves AW-500, containing about 65 wt.% $SiO_2$ and about 23 wt.% $Al_2O_3$, or a $SiO_2/Al_2O_3$ molar ratio of about 4.8, and having a pore size of about 5 Å; and AW-300, having a pore size of about 4 Å. Another commercially available activated molecular sieve is the Linde LZ-Y52, containing about 64 wt.% $SiO_2$ and about 23 wt.% $Al_2O_3$, or a $SiO_2/Al_2O_3$ molar ratio of about 4.75, and having a pore size of about 8 Å. Preferably, the molecular sieves have a $SiO_2/Al_2O_3$ molar ratio greater than about 4.5.

In a preferred embodiment, the catalyst of the present inventive process is prepared by sulfate-activating molecular sieves by treatment with a sulfate-containing compound. Preferably, the sulfate-containing compound is selected from the group consisting of ammonium sulfate, ammonium hydrogen sulfate, sulfuric acid, sulfur trioxide, sulfur dioxide and hydrogen sulfide. Especially preferred sulfating agents are ammonium sulfate and sulfuric acid. Said agents may be employed neat, or as an aqueous, ketonic, alcoholic, or ether solution, but preferably as an aqueous solution. Said sulfating agents also may be employed as mixtures of the sulfating agents listed above. Excess sulfating agent may be removed by filtration or evaporation.

Preferably, the sulfate-activated molecular sieves are then calcined prior to use as an oligomerization catalyst. Calcination in air or in an inert gas environment, such as nitrogen, may be conducted at a temperature of at least 100°C, but below the temperature at which thermal destruction leads to catalyst deactivation. The optimal temperature range can be determined by routine experimentation for a particular type of molecular sieve. Typically, the sulfated molecular sieves are calcined for about 1 to 24 hours at a temperature of from about 500° to 800° C. Temperatures above 900° C should be avoided.

In a more specific embodiment, crystalline aluminosilicate molecular sieves are sulfate-activated by adding ammonium sulfate neat or, if desired, diluted with distilled water, to the molecular sieves. The slurry is then mixed for about 1 to 24 hours, filtered, washed, and calcined in a stream of air for about 1 to 24 hours. The weight percent of ammonium sulfate to crystalline aluminosilicate molecular sieve should be such that the concentration of the sulfur in the formulated catalyst (before calcination) is in the range of about 0.1 wt.% to 30 wt.%, although concentrations outside this range also may be employed.

In a further embodiment, the sulfate-activated catalyst may be prepared by treating a Group IV oxide with a sulfate containing compound. Preferably, the sulfate-containing compound is selected from those compounds described above.

Preferably, the sulfated Group IV oxide is then calcined prior to use as an oligomerization catalyst, as described above.

Good results were achieved, for example, for the ammonium sulfate on zirconia catalyst by calcining at 625°-750°C, for 15 hours, in a stream of nitrogen.

Suitable Group IV oxides used in conjunction with said sulfur-containing compounds include, for example,

the oxides of silicon, titanium, zirconium, hafnium, germanium, tin, and lead, as well as combinations thereof. Particularly preferred are oxides of titanium and zirconium, such as the anatase or rutile forms of titania and zirconia.

In a more specific embodiment, the Group IV oxide is treated with sulfuric acid by adding said acid neat or, if desired, diluted with distilled water, to the oxide extrudates. The slurry is then mixed for about 1 to 24 hours, filtered, washed, and calcined in a stream of air for about 1 to 24 hours. The prepared sulfuric acid-treated oxide should then have a titratable acidity of at least 0.1 meq/g.

The weight percent of sulfuric acid to Group IV oxide should be such that the concentration of the sulfur in the formulated catalyst is in the range of about 0.1 wt. % to 30 wt. %, although concentrations outside this range also may be employed.

Generally, the catalyst composition is prepared by impregnating a pre-formed pellet, extrudate or powder. A suitable procedure to be used is to immerse catalyst pellets, for example, in an aqueous or polar organic solvent solution of the acid, preferably at ambient temperature. Higher temperatures of about 100° C to about 150° C may be used, if desired. This treatment should be continued, preferably with agitation, for about 0.1 to about 5 hours. The conditions should be sufficient to permit the solution to penetrate the pores of the catalyst pellet. The amount of acid solution that is used should be adequate to permit full immersion of the catalyst pellets. Larger amounts of the solution can be used, if desired, but there is no particular advantage in doing so. At the end of the immersion step, the excess solution can be evaporated from the treated pellets, or the pellets can be removed from the solution and permitted to dry (e.g., in a drying oven).

The catalysts to be activated may be in the form of powders, pellets, spheres, shapes and extrudates. For example, titania pellets may be prepared by extrusion or by compaction in conventional pelleting apparatus using a pelleting aid such as graphite. Extrudates which work well include HSA titania carrier extrudate from Norton Company, with a surface area of $51 m^2/g$, and zirconia extrudates from Norton having a surface area of $77 m^2/g$.

Cylindrically-shaped catalyst pellets having a diameter essentially equal to the length thereof can be employed. Diameters ranging from about 0.794 mm (1/32 inch) to about 9.525 mm (3/8 inch) possess desirable dimensions. The shape and dimensions of the pellets are not critical to the present invention; pellets of any suitable shape and dimensions may be used.

When cylindrical pellets of catalyst of the type described above are used, the liquid hourly space velocity may be varied within wide limits (e.g., 0.1 to 10) in order to obtain a desired rate of conversion. Normally, space velocities of about 0.5 to 2 LHSV will be employed.

Preferably, the pelleted catalyst compositions used in the present inventive process are employed as a fixed bed of catalyst in a continuous reaction system. In a continuous process of this nature, the time of contact of the reactants with the catalyst is one of the interrelated factors that those skilled in the art will adjust, along with temperature, pressure, bed geometry, pellet size, etc., in order to obtain a desired rate of reaction and, hence, a desired percentage of conversion of the reactants. In a continuous process, it is not necessary to drive the reaction to completion, because unreacted feedstock components may be recycled to the reactor.

The catalyst compositions of the present invention are advantageously used in a continuous process for the continuous production of oligomers from long-chain olefins. Such catalyst compositions can be used for prolonged periods without the need for regeneration. Nevertheless, with the passage of time, deactivation will tend to slowly occur. Deactivation can be measured qualitatively by the loss of olefin conversion, or as the increase of temperature required to maintain an essentially constant conversion rate for the olefin.

As an alternative approach for the preparation of the Group IV activated catalysts used in the present invention process, a sulfate-activated Group IV oxide may be prepared by a one-step process, comprising heating a compound such as titanium sulfate hydrate at a temperature in the range of about 500°C to about 625°C. Sulfate-activated zirconium dioxide compounds may be prepared in a similar manner using, for example, zirconium sulfate hydrate.

The oligomerization reaction may be carried out either batchwise, in a stirred slurry reactor, or continuously, in a fixed bed continuous flow reactor. The catalyst concentration should be sufficient to provide the desired catalytic effect. The temperatures at which the oligomerization may be performed are between about 50 and 300° C, with the preferred range being about 120 to 250° C, and the especially preferred range being about 160 to 180°C, for optimum conversion. At temperatures of about 200°C or greater, the amount of unsaturation remaining in the products of the oligomerization reaction may decrease, thus reducing the degree of hydrogenation necessary to remove unsaturation from the base stocks. However, at temperatures above 200°C, the olefin conversion may decrease. The dimer to trimer ratio may increase. It has been found that the addition of a hydrocarbon containing a tertiary hydrogen, such as methylcyclohexane, may further reduce the amount of unsaturation present in the base stocks. The reaction conditions may be chosen to be most suited to the results desired for a particular application. The reaction may be run at pressures of from 0 to 6895kPa gp (1000 psig).

Following the oligomerization reaction, the unsaturated oligomers may be hydrogenated to improve their thermal stability and to guard against oxidative degradation during their use as lubricants. The hydrogenation reaction for 1-decene oligomers may be represented as follows:

$$C_{10n}H_{20n} + H_2 \xrightarrow{\text{catalyst}} C_{10n}H_{(20n+2)}$$

where n represents moles of monomer used to form the oligomer. Known hydrogenation processes may be used to hydrogenate the oligomers. A number of metal catalysts are suitable for promoting the hydrogenation reaction, including nickel, platinum, palladium, copper, and Raney nickel. These metals may be supported on a variety of porous materials such as kieselguhr, alumina, or charcoal, or they may be formulated into a bulk metal catalyst. A particularly preferred catalyst for this hydrogenation is a nickel-copper-chromia catalyst described in U.S. Patent No. 3,152,998, incorporated by reference herein. Other U.S. patents disclosing known hydrogenation procedures include U.S. Patent Nos. 4,045,508; 4,013,736; 3,997,622; and 3,997,621.

Unreacted monomer may be removed either prior to or after the hydrogenation step. Optionally, unreacted monomer may be stripped from the oligomers prior to hydrogenation and recycled to the catalyst bed for oligomerization. The removal or recycle of unreacted monomer or, if after hydrogenation, the removal of non-oligomerized alkane, should be conducted under mild conditions using vacuum distillation procedures known to those skilled in the art. Distillation at temperatures exceeding 250° C may cause the oligomers to break down in some fashion and come off as volatiles. Preferably, therefore, the reboiler or pot temperature should be kept at or under about 225° C when stripping out the monomer. Procedures known by those skilled in the art to be alternatives to vacuum distillation also. may be employed to separate unreacted components from the oligomer.

While it is known to include a distillation step after the hydrogenation procedure to obtain products of various 100° C viscosities, it is preferred in the method of the present invention that no further distillation (beyond monomer flashing) be conducted. In other words, the monomer-stripped, hydrogenated bottoms are the desired synthetic lubricant components. Thus, the method of this invention does not require the costly, customary distillation step, yet, surprisingly, produces a synthetic lubricant component that has excellent properties and that performs in a superior fashion. However, in some contexts, one skilled in the art may find subsequent distillation useful in the practice of this invention.

The invention will be further illustrated by the following non-limiting examples.

## EXAMPLES

In the examples detailed in Table I below, the following procedures were used:

### Preparation of Catalysts

Titanium dioxide or zirconium dioxide extrudates (3.17mm) were charged to a crucible and covered with 10 % ammonium sulfate solution. The pellets were allowed to stand for 15 minutes, then placed in an oven (with nitrogen purge) and heated slowly to the desired temperature for the desired time. The pellets were then cooled to less than 200° C and placed in a tightly sealed glass bottle until just before use.

### Oligomerization of Olefins

The catalyst pellets prepared above were ground to a fine powder. Olefin and catalyst were charged to a flask equipped with, an overhead stirrer, thermometer, heating mantle, and a water-cooled condenser ($N_2$ purge). The mixture was vigorously stirred and heated to the desired temperature for the desired time. The mixture was then cooled to ambient temperature and filtered with suction. The liquid was analyzed by liquid chromatography. The results obtained are detailed in Table I.

TABLE I

USE OF PELLETED GROUP IV OXIDES ACTIVATED WITH SULFUR-CONTAINING COMPOUNDS TO OLIGOMERIZE -OLEFINS

| Ex. No. | Olefin (by carbon number) | (g) of Olefin | Catalyst | Amount of Catalyst (g) | Time/Temp. (Hr)/(°C) | Olefin Con. (%) | D/T+ Ratio |
|---|---|---|---|---|---|---|---|
| 1 * | C-14 α | 100 | $TiO_2$ [a] | 10 | 5/160 | ~0 | - |
| 2 * | C-14 α | 100 | $TiO_2$ [a] | 10 | 4/180 | ~0 | - |
| 3 * | C-14 α | 100 | $TiO_2$ [b] | 10 | 5/160 | ~0 | - |
| 4 * | C-14 α | 100 | $TiO_2$ [b] | 10 | 4/180 | 12.6 | - |
| 5 | C-14 α | 100 | $TiO_2$ [c] | 10 | 5/160 | 2.0 | - |
| 6 | C-14 α | 100 | $TiO_2$ [d] | 10 | 5/160 | 64.6 | 4.27 |
| 7 * | C-14 α | 100 | $ZrO_2/SiO_2$ [e] | 10 | 5/160 | ~0 | - |
| 8 * | C-14 α | 100 | $ZrO_2$ [f] | 10 | 5/160 | ~0 | - |
| 9 | C-14 α | 100 | $ZrO_2$ [g] | 10 | 5/160 | 86.8 | 1.68 |
| 10 | C-14 α | 100 | $TiO_2$ [h] | 10 | 5/160 | 31.0 | 12.4 |
| 11 | C-14 α | 100 | $ZrO_2$ [g] | 10 | 4/180 | 78.6 | 2.32 |
| 12 | C-14 α | 100 | $TiO_2$ [h] | 10 | 4/180 | 36.9 | 9.28 |
| 13 | C-10 α | 100 | $ZrO_2$ [g] | 10 | 4/140 | 88.0 | 2.30 |
| 14 | C-10 α | 100 | $ZrO_2$ [g] | 10 | 4/120 | 52.6 | 6.47 |
| 15 | C-10 α | 100 | $ZrO_2$ [g] | 10 | 4/160 | 90.4 | 1.53 |
| 16 | C-14 α | 100 | $ZrO_2$ [i] | 10 | 5/160 | 75.0 | 3.19 |

Con. = Conversion; D = Dimer; and T+ = Trimer + Tetramer + Pentamer, etc.

[a] Norton $TiO_2$ "as-is."
[b] $TiO_2$ calcined at 625° C for 15 hours.
[c] $TiO_2$ treated with sulfuric acid and calcined at 300° C.
[d] $TiO_2$ treated with sulfuric acid and calcined at 625° C.
[e] $ZrO_2/SiO_2$ calcined at 625° C for 15 hours.
[f] $ZrO_2$ calcined at 625° C for 15 hours.
[g] $ZrO_2$ treated with 10% ammonium sulfate and heated at 625° C for 15 hours.
[h] $TiO_2$ treated with 10% ammonium sulfate and heated at 625° C for 15 hours.
[i] $ZrO_2$ treated with ammonium sulfate and heated at 750° C for 15 hours.

* - Not in accordance with the present invention.

In the examples detailed in Table II below, the following procedures were used:

One-Step Preparation of Catalysts

Titanium sulfate hydrate was calcined at 625° C for 15 hours. Zirconium sulfate hydrate was treated in a similar manner.

Oligomerization of Olefins

Olefin and catalyst were charged to a flask equipped with an overhead stirrer, thermometer, heating mantle, and a water-cooled condenser ($N_2$ purge). The mixture was vigorously stirred and heated to the desired temperature for the desired time. The mixture was then cooled to ambient temperature and filtered with suction. The liquid was analyzed by liquid chromatography. The results obtained are detailed in Table II.

TABLE II

| Ex. No. | Olefin(s) (by carbon number) | (g) of Olefin | Catalyst | Amount of Catalyst (g) | Time/Temp. (Hr)/(°C) | Olefin Con. (%) | D/T+ Ratio |
|---|---|---|---|---|---|---|---|
| 1* | C-14 α | 100 | $Ti(SO_4)_2 \cdot H_2O$[1] | 10 | 5/160 | 0.00 | - |
| 2* | C-14 α | 100 | $Ti(SO_4)_2 \cdot H_2O \cdot H_2SO_4$[1] | 10 | 5/160 | ~2 | - |
| 3 | C-14 α | 100 | $TiO_2$[2] | 10 | 5/160 | 36.4 | 14.3 |
| 4 | C-14 α | 100 | $TiO_2$[2] | 10 | 4/180 | 49.2 | 11.0 |
| 5 | C-10 α | 100 | $TiO_2$[2] | 10 | 5/160 | 64.0 | 5.57 |
| 6 | C-10 α | 100 | $TiO_2$[2] | 10 | 4/180 | 81.1 | 3.01 |
| 7 | C-1314i | 100 | $TiO_2$[2] | 10 | 4/180 | 37.6 | - |
| 8 | C-14 α | 84 | $ZrO_2$[3] | 8 | 5/160 | 85.9 | 1.81 |

Con. = Conversion; D = Dimer; and T+ = Trimer + Tetramer + Pentamer, etc.

[1] Not calcined.

[2] Prepared by heating titanium sulfate hydrate at 625 °C for 15 hours.

[3] $Zr(SO_4)_2 \cdot xH_2O$ calcined at 625 °C.

* Not in accordance with the present invention.

A series of tests were then carried out using molecular sieves:

Catalysts

Catalyst 1 - Aldrich molecular sieve LZ-Y52 pellets (1/16") "as is," i.e., non sulfate-activated (1/16" is equivalent to 1.6mm)

Catalyst 2 - Aldrich molecular sieve LZ-Y52 pellets (1/16") were placed in a crucible and covered with 10 % ammonium sulfate solution. The crucible was then placed in an oven and heated to 400° C and held at that temperature for 23 hours. A nitrogen purge was used. The sulfate-activated molecular sieves were then cooled to ambient temperature (under nitrogen), and placed in a stoppered bottle until use.

Catalyst 3 - Aldrich molecular sieve AW-500 pellets (1/16") were treated according to the procedure used for Catalyst 2, above.

Catalyst 4 - Aldrich molecular sieve 4A beads (1/16") were treated according to the procedure used for Catalyst 2, above.

Catalyst 5 - Aldrich molecular sieve AW-300 beads (1/16") "as is," i.e., non sulfate-activated.

Catalyst 6 - Aldrich molecular sieve AW-300 beads (1/16") were placed in a crucible. The crucible was then placed in an oven and heated to 500° C and held at that temperature for 19 hours. A nitrogen purge was used. The molecular sieves were then cooled to ambient temperature (under nitrogen), and placed in a stoppered bottle until use. No ammonium sulfate was used.

Catalyst 7 - Aldrich molecular sieve AW-300 beads (1/16") were placed in a crucible and covered with 10 % ammonium sulfate solution. The crucible was then placed in an oven and heated to 500° C and held at that temperature for 19 hours. A nitrogen purge was used. The sulfate-activated molecular sieves were then cooled to ambient temperature (under nitrogen), and placed in a stoppered bottle until use.

Procedure: Oligomerization of Olefins

The catalyst pellets and beads described above were ground to a fine powder. Olefin and catalyst were charged to a flask equipped with an overhead stirrer, thermometer, heating mantle, and a water-cooled condenser ($N_2$ purge). The mixture was vigorously stirred and heated to the desired temperature for the desired time. The mixture was then cooled to ambient temperature and filtered with suction. The liquid was analyzed by liquid chromatography. The results obtained are detailed in the table below.

| Ex. No. | Olefin (by carbon number) | (g) of Olefin | Catalyst | Amount of Catalyst (g) | Time/Temp. (Hr)/(°C) | Olefin Con. (%) | M (%) | D (%) | T+ (%) | D/T+ Ratio |
|---|---|---|---|---|---|---|---|---|---|---|
| 17 | C-14 α | 100 | Cat. 4: 4A (SA) | 10 | 5/160 | 3.0 | 97.0 | 3.0 | - | - |
| 18 | C-14 α | 100 | Cat. 3: AW-500 (SA) | 10 | 5/160 | 43.1 | 56.9 | 35.8 | 7.27 | 4.92 |
| 19* | C-14 α | 100 | Cat. 1: LZ-Y52 | 10 | 4/180 | 18.8 | 81.2 | 18.8 | - | - |
| 20 | C-14 α | 100 | Cat. 2: LZ-Y52 (SA) | 10 | 5/160 | 15.7 | 84.3 | 15.7 | - | - |
| 21 | C-14 α | 100 | Cat. 2: LZ-Y52 (SA) | 10 | 4/180 | 32.5 | 67.5 | 32.5 | - | - |
| 22 | C-14 α | 100 | Cat. 3: AW-500 (SA) | 10 | 4/180 | 48.0 | 52.0 | 41.9 | 6.10 | 6.87 |
| 23 | C-14 α | 100 | Cat. 4: 4A (SA) | 10 | 4/180 | 11.3 | 88.7 | 11.3 | - | - |
| 24* | C-14 α | 100 | Cat. 5: AW-300 | 10 | 5/160 | 19.3 | 80.7 | 16.9 | 2.41 | 7.01 |
| 25* | C-10 α | 100 | Cat. 5: AW-300 | 10 | 5/160 | 32.5 | 67.5 | 25.7 | 4.72 | 5.44 |
| 26* | C-14 α | 100 | Cat. 6: AW-300 (Calcined only) | 10 | 5/160 | 21.4 | 78.6 | 18.8 | 2.61 | 7.20 |
| 27* | C-10 α | 100 | Cat. 6: AW-300 (Calcined only) | 10 | 5/160 | 17.7 | 82.3 | 15.5 | 2.19 | 7.08 |
| 28 | C-14 α | 100 | Cat. 7: AW-300 (SA) | 10 | 5/160 | 56.2 | 43.8 | 43.5 | 12.80 | 3.40 |
| 29 | C-10 α | 100 | Cat. 7: AW-300 (SA) | 10 | 5/160 | 70.4 | 29.6 | 50.0 | 20.30 | 2.46 |

Con. = Conversion; M = Monomer; D = Dimer;

and T+ = Trimer + Tetramer + Pentamer, etc.; SA = Sulfate-activated.

* Not in accordance with the present invention.

## Claims

1. A process for the preparation of oligomers comprising oligomerizing a linear olefin containing from 10 to 24 carbon atoms in the presence of a catalyst, characterised in that said catalyst is a sulfate-activated Group IV oxide or molecular sieve.

2. A process as claimed in Claim 1 wherein the sulfate-activated Group IV oxide or molecular sieve is prepared by a two-step method comprising (1) treating a Group IV oxide or molecular sieve with a sulfate-containing compound and (b) calcining the treated Group IV oxide or molecular sieve at a temperature of

500-800°C.

3. A process as claimed in Claim 1 or Claim 2 wherein the linear olefins comprise alpha-olefins, internal olefins or a mixture thereof.

4. A process as claimed in any one of Claims 1 to 3 wherein the olefin composition comprises said linear olefins and up to 20 weight % of propylene.

5. A process as claimed in any one of Claims 1 to 3 wherein the olefin composition comprises said linear olefins and up to 20 weight % of 1,3-diisopropenyl benzene.

6. A process as claimed in any one of Claims 1 to 3 wherein the olefin composition comprises said linear olefins and a vinylidene olefin having 10 to 24 carbon atoms.

7. A process as claimed in any one of Claims 1 to 6 wherein oligomerization is carried out at a temperature in the range of 50°C to 300°C and at a pressure of atmospheric to 6985kPa gauge pressure (1000 psig).

8. A process as claimed in any one of Claims 1 to 7 wherein sulfate activation is carried out by treatment with a sulfate-containing compound selected from ammonium sulfate, ammonium hydrogen sulfate, sulfuric acid, sulfur trioxide, sulfur dioxide and hydrogen sulfide.

9. A process as claimed in Claim 1 or Claims 3 to 8 wherein the sulfate-activated Group IV oxide is prepared by a one-step method comprising heating titanium sulfate hydrate at a temperature greater than about 500°C.

10. A process as claimed in any one of Claims 1 to 9 wherein the Group IV oxide is titanium dioxide or zirconium dioxide.

11. A process as claimed in any one of Claims 1 to 8 wherein the molecular sieves are crystalline aluminosilicate molecular sieves.

12. A process as claimed in Claim 11 wherein the crystalline aluminosilicate molecular sieves have a $SiO_2/Al_2O_3$ molar ratio greater than about 4.5.

13. A process as claimed in Claim 11 or Claim 12 wherein the molecular sieve has enhanced Bronsted acidity obtained by acid wash or hydrolysis or by treatment with an ammonium-ion containing compound, followed by decomposition of the ammonium ion-exchanged form.

14. A process as claimed in any one of Claims 1 to 13, wherein any unreacted olefin is separated from the oligomerized product, and the oligomers are hydrogenated.

European Patent Office

# EUROPEAN SEARCH REPORT

Application Number
EP 92 30 9842

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | GB-A-1 113 193 (THE BRITISH PETROLEUM COMPANY)<br>* page 1, line 27 - page 1, line 42 *<br>* page 1, line 68 - page 1, line 75 *<br>--- | 1-3,11 | C07C2/16<br>B01J37/00 |
| A | US-A-4 423 267 (DOWLING ET AL)<br>* claims; examples *<br>--- | 1-2,14 | |
| A | FR-A-2 641 477 (INSTITUT FRANCAIS DU PETROLE)<br>--- | | |
| A,D | EP-A-0 449 453 (TEXACO CHEMICAL COMPANY)<br>--- | | |
| P,X | US-A-5 113 034 (SOLED ET AL)<br>* examples *<br>----- | 1-3 | |

TECHNICAL FIELDS SEARCHED (Int. Cl.5 )

C07C
C10G

The present search report has been drawn up for all claims

| Place of search | Date of completion of the search | Examiner |
|---|---|---|
| THE HAGUE | 06 JANUARY 1993 | J. VAN GEYT |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)